# EUROPÄISCHE PATENTSCHRIFT

(11) **EP 0 645 161 B1**
(45) Veröffentlichungstag und Bekanntmachung des Hinweises auf die Patenterteilung: **06.02.2002**
(21) Anmeldenummer: 94115242.3
(22) Anmeldetag: 28.09.1994
(51) Int. Cl.: A61M 39/10, A61M 39/12, F16L 33/00, F16L 31/00

(54) **Schlauchverbinder**
Tube connector
Raccord pour tube

(30) Priorität: 29.09.1993 DE 4333070
(43) Veröffentlichungstag der Anmeldung: 29.03.1995
(73) Patentinhaber: B. BRAUN MELSUNGEN AG, 34212 Melsungen (DE)
(72) Erfinder: Mehner, Gotthilf, D-66280 Sulzbach (DE); Ludt, Peter, D-66271 Bliesransbach (DE); Eick, Rüdiger, D-66459 Kirkel (DE)
(74) Vertreter: Selting, Günther, Dipl.-Ing.

(56) Entgegenhaltungen:
- EP-A- 0 123 761
- DE-A- 2 738 108
- DE-A- 2 949 315
- DE-U- 7 823 414
- US-A- 3 777 354
- US-A- 4 822 078

## Beschreibung

### Technisches Gebiet

Die Erfindung betrifft Schlauchverbinder aus gummielastischem Material zum Verbinden eines Schlauches mit in den Schlauchverbinder einzusteckenden dünneren Schläuchen unterschiedlichen Durchmessers mit abtrennbaren Kupplungsabschnitten mit stufenförmig sich erweiterndem Lumen.

### Stand der Technik

Derartige Schlauchverbinder sind in der Medizintechnik vielfach in Gebrauch. Sie dienen zum Verbinden eines Schlauches, mit dessen freiem Ende sie fest verbunden sind, mit Schläuchen geringeren Durchmessers. Aus DE-GM 7823414 ist ein Schlauchverbinder bekannt, dessen Lumen sich stufenförmig in Richtung auf den zu verbindenden Schlauch verengt. In der Regel ist jeweils auf der Außenseite der so entstehenden Kupplungsabschnitte der Durchmesser des in das Lumen dichtend einschiebbaren Schlauches angegeben. Meist wird das Lumen in dem in der Medizin üblichen Maß Charrière (Ch) angegeben. Je nach Durchmesser des einzuschiebenden Schlauches werden die Kupplungsabschnitte mit zu kleinem inneren Durchmesser abgetrennt. Es ist üblich, die Kupplungsabschnitte außen entsprechend den Stufen des Lumens zu markieren, um dem Benutzer das Durchtrennen des Schlauchverbinders an der richtigen Stelle zu ermöglichen. Die Markierung erfolgt z. B., indem auch die Außenseite des Schlauchverbinders entsprechend dem stufenförmigen Verlauf des Lumens sich stufenförmig verengend verläuft. Zusätzlich oder alternativ können die Stufen des Lumens außen durch ringförmige Markierungen, beispielsweise durch umlaufende Ringnute gekennzeichnet werden.

Diese Schlauchverbinder werden z. B. verwendet, um einen von einer Saugeinrichtung, wie z. B. einer Redon-Saugflasche, kommenden relativ dicken Schlauch mit dem patientenfernen Ende eines in eine Operationswunde eingelegten dünneren Saugdrains zu verbinden. Es versteht sich von selbst, daß das Verbinden der Schläuche mit möglichst geringem Kraftaufwand möglich sein sollte, deren Lösen jedoch eine möglichst hohe Zugkraft erfordern soll.

### Beschreibung der Erfindung

Ein Ziel der vorliegenden Erfindung ist demnach die Zurverfügungstellung eines Schlauchverbinders, der ein zugresistentes Verbinden mit geringem Kraftaufwand erlaubt.

Gelöst wird die Aufgabe mit den Merkmalen des Patentanspruchs 1.

Die Ringwulste in den zylindrischen Kupplungsabschnitten sind vorzugsweise in Richtung auf das weitere Ende des Schlauchverbinders, das heißt nach patientenfern, geneigt, wobei der Neigungswinkel bezogen auf die Senkrechte der Schlauchachse zwischen 10 und 45 Grad liegt. Der in den Schlauchverbinder einzuschiebende Schlauch läßt sich durch diese Maßnahme gegen einen sehr geringen Widerstand in das Lumen des passenden Kupplungsabschnittes einschieben. Beim Herausziehen versuchen sich die geneigten Ringwulste aufzustellen, wobei sich die patientenfernen Kanten der Ringwulste in die Außenseite des zu fixierenden Schlauches widerhakenartig eingraben und somit ein Dekonnektieren sehr erschweren.

Entsprechend leicht läßt sich ein zu verbindender Schlauch in die Kupplungsabschnitte mit konischen Bohrungen einschieben. Der zu verbindende Schlauch wird über die Schulter am Übergang zum nächsten Kupplungsabschnitt hinaus eingeschoben. Wie vorstehend im Zusammenhang mit den Ringwulsten erläutert, wird die Schulter am Übergang zum nächsten Kupplungsabschnitt hinaus eingeschoben. Wie vorstehend im Zusammenhang mit den Ringwulsten erläutert, wirkt die Schulter am Übergang zum nächsten Kupplungsabschnitt beim Zurückziehen widerhakenartig unter Verstärkung des Formschlusses auf den eingeschobenen Schlauch und verhindert damit ein unbeabsichtigtes Lösen der Schlauchverbindung.

Für die erfindungsgemäßen Schlauchverbinder ergeben sich verschiedene Gestaltungsmöglichkeiten. So können Schlauchverbinder zur Verfügung gestellt werden, die ausschließlich Kupplungsabschnitte umfassen, deren zylindrische Lumina jeweils einen oder mehrere Ringwulste aufweisen. Alternativ weisen sämtliche Kupplungsabschnitte konisch verlaufende Lumina auf. In einer besonderen Ausführungsform weisen die Schlauchverbinder Kupplungsabschnitte beider Gestaltungsweisen auf. Hierbei ist es sinnvoll, eine erste zusammenhängende Gruppe von Kupplungsabschnitten nach der einen Art und eine zweite Gruppe von zusammenhängenden Kupplungsabschnitten nach der zweiten Art zu gestalten. Vorzugsweise werden die Kupplungsabschnitte mit kleineren Innenweiten mit Ringwulsten ausgestattet und die Kupplungsabschnitte mit größeren Innenweiten konisch ausgeführt.

Die in der Medizintechnik verwendeten Schläuche weisen in der Regel Weiten von 6 bis 18 Charrière auf, wobei die Charrière-Zahlen praktisch durchweg geradzahlig sind. Um den gesamten Bereich abzudecken, weisen erfindungsgemäße Schlauchverbinder sieben Kupplungsabschnitte mit den verschiedenen Weiten für Schläuche von 6, 8, 10, 12, 14, 16 und 18 Charrière auf. Die Kupplungsabschnitte sind durch die entsprechenden Charrière-Werte gekennzeichnet. Vorzugsweise sind die Trennstellen zwischen den Kupplungsabschnitten durch umlaufende Nuten gekennzeichnet. Soll beispielsweise der Schlauchverbinder zum Verbinden mit einem Schlauch der Weite 12 Charrière vorbereitet werden, so wird der Schlauchverbinder zwischen dem mit "10" und dem mit "12" markierten Kupplungsabschnitt durchtrennt.

Es ist auch möglich, die Zahl der Kupplungsabschnitte zu verringern, indem Kupplungsabschnitte vorgesehen werden, die aufgrund der Dehnbarkeit des Schlauchverbinders für zwei benachbarte Charrière-Weiten geeignet sind.

Die Materialien, aus denen erfindungsgemäße Schlauchverbinder hergestellt werden können, sowie die Verfahren zur Herstellung erfindungsgemäßer Schlauchverbinder entsprechen dem Stand der Technik und sind dem Fachmann bekannt.

Anhand der Zeichnungen werden Ausführungsbeispiele der Erfindung wie folgt beschrieben.

Es zeigen:
- Fig. 1: eine Außenansicht eines ersten Ausführungsbeispiels eines Schlauchverbinders;
- Fig. 2: den Schlauchverbinder nach Fig. 1 im Längsschnitt und zwar eine Ausführung mit sich stufenförmig erweiternden Bohrungsabschnitten, deren Lumina zylindrisch sind und Ringwulste aufweisen;
- Fig. 3: die Anwendung des Schlauchverbinders nach Fig. 1 und 2;
- Fig. 4: einen vergrößerten Detailschnitt aus dem Bereich IV der Fig. 3;
- Fig. 5: ein weiteres Ausführungsbeispiel eines Schlauchverbinders mit sich stufenförmig erweiternden Abschnitten, deren Lumina konisch sind und welche damit endseitig Ringschultern bilden;
- Fig. 6: die Anwendung des Schlauchverbinders nach Fig. 5;
- Fig. 7: ein drittes Ausführungsbeispiel eines Schlauchverbinders, welcher sowohl zylindrische Lumina mit Ringwulsten nach Fig. 2 als auch konische Lumina mit endseitigen Ringschultern nach Fig. 5 aufweist;
- Fig. 8: die Anwendung des Schlauchverbinders nach Fig. 7 für Schläuche mit kleinen Durchmessern;
- Fig. 9: die Anwendung des Schlauchverbinders nach Fig. 7 für größere Schlauchdurchmesser.

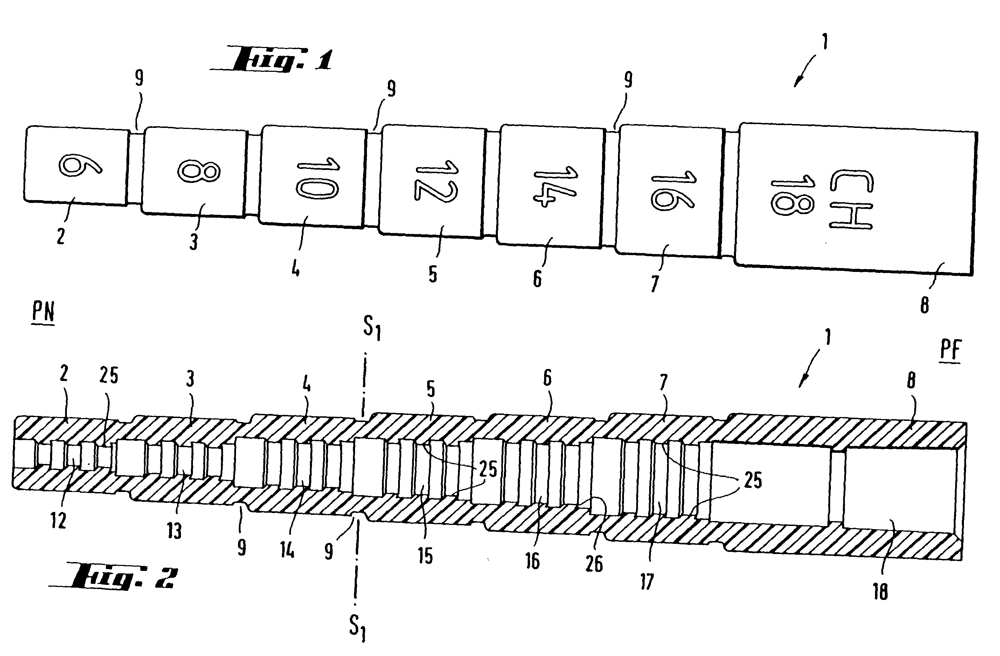

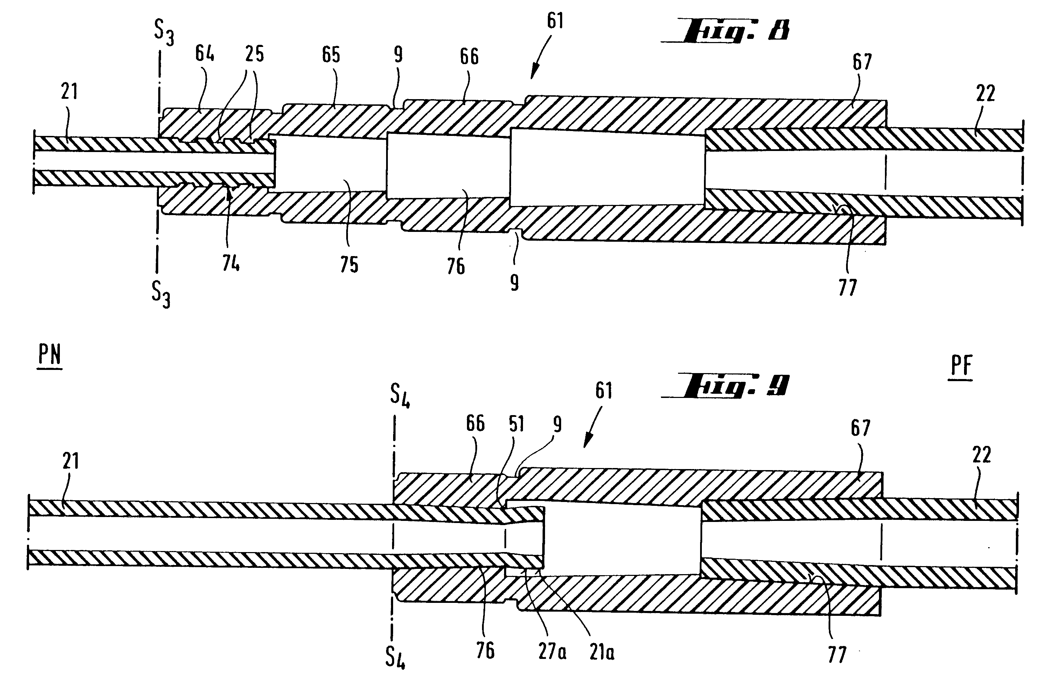

Der Schlauchverbinder 1 der Figuren 1 bis 4 besteht aus sieben Kupplungsabschnitten 2 bis 8, welche durch Nuten 9 begrenzt sind. Jedem Kupplungsabschnitt 2 bis 8 ist ein zylindrischer Bohrungsabschnitt 12 bis 18 zugeordnet und zwar dermaßen, daß sich deren Lumen von der patientennahen Seite PN bis zu patientenfernen Seite PF stufenförmig erweitert.

Der patientenferne Saugschlauch 22 wird in an sich bekannter Weise im konischen Bohrungsabschnitt 18 des Kupplungsabschnittes 8 befestigt.

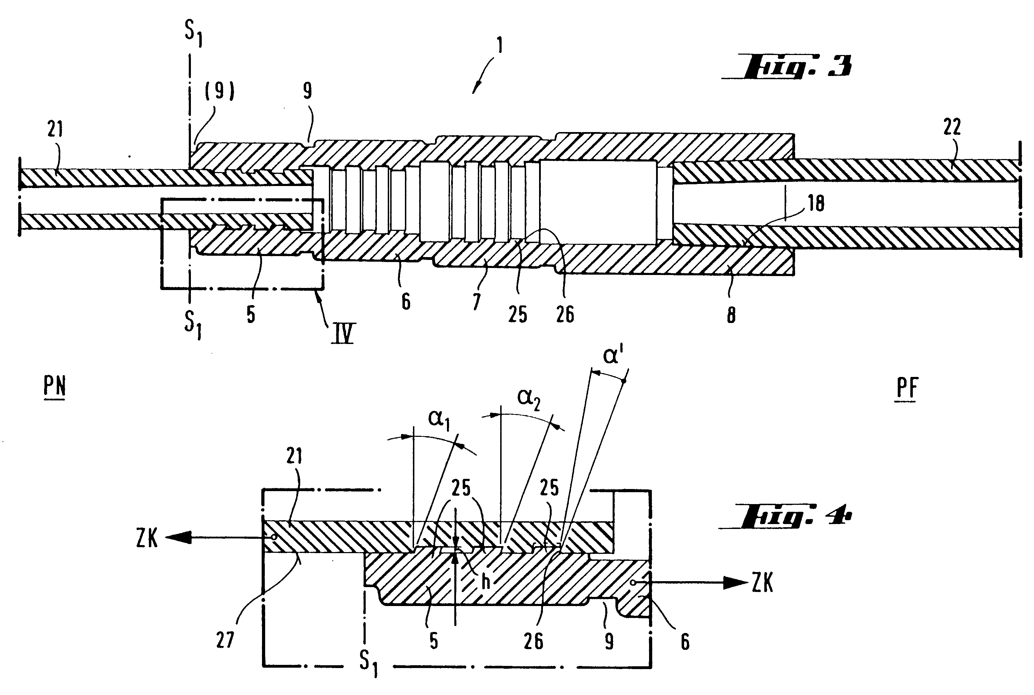

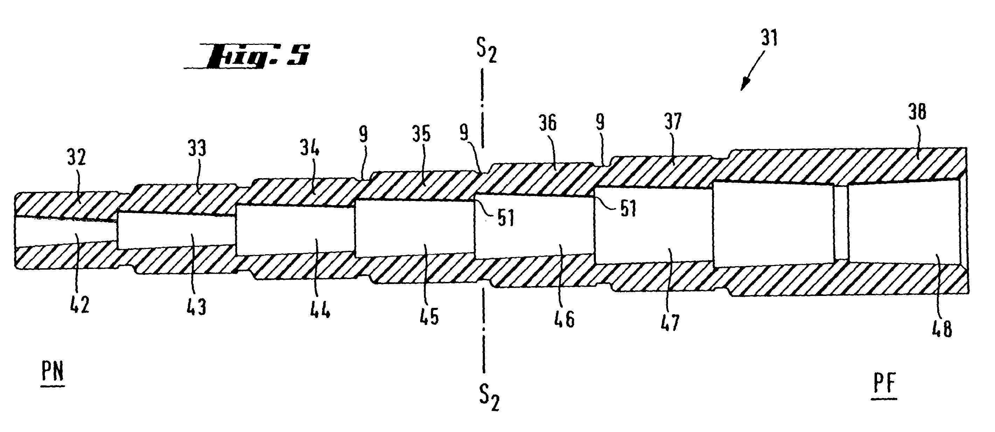

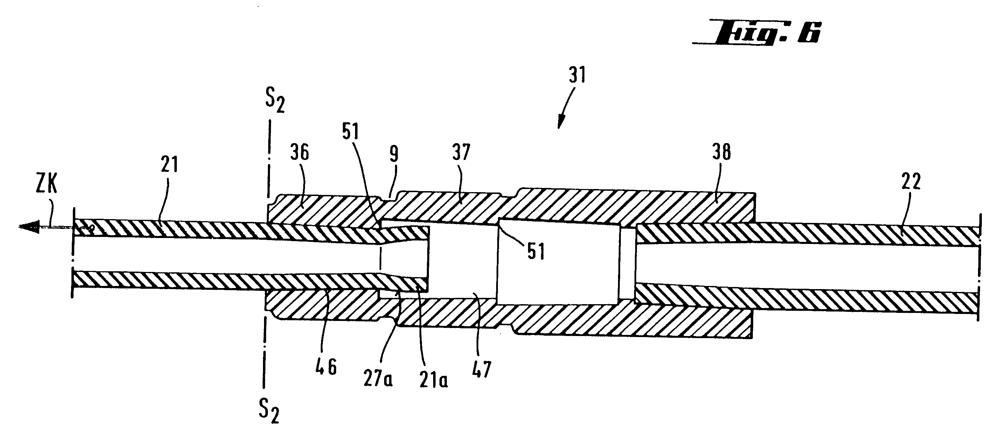

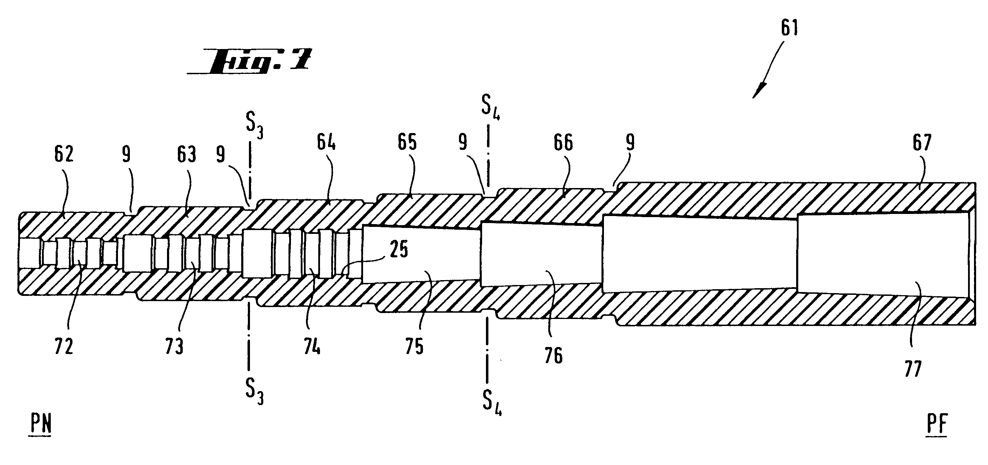

Der patientennahe Saugschlauch 21 wird je nach den hydraulischen Erfordernissen lumenmäßig (Charrière) ausgewählt und in den dazugehörigen zylindrischen Bohrungsschnitt 12 bis 17 eingeführt. Erreicht wird dies, indem die zu kleinen Kupplungsabschnitte, in Fig. 3 z. B. die Abschnitte 2, 3 und 4, durch Abschneiden an der Schnittstelle S entfernt werden. Als Hilfe für die zutreffenden Abschneidemaßnahmen sind zum einen die Charrière-Angaben CH 6 bis CH 18 (vgl. Fig. 1) auf der Außenseite des Schlauchverbinders 1 markiert und zum anderen zwischen den Abschnitten 2 bis 7 Schneidmarkierungen in Form von umlaufenden Nuten 9 vorgesehen.

Um das Herausziehen des Schlauches 21 aus dem Schlauchverbinder 1 zu erschweren bzw. dessen Halt zu optimieren sind erfindungsgemäß in den zylindrischen Bohrungsabschnitten umlaufende Ringwulste 25 angeordnet.

Vorzugsweise sind diese Ringwulste 25, ebenfalls erfindungsgemäß, an ihrer patientennahen Seite PN um einen Winkel α₁ von ca. 10 bis 45 Grad in Einschubrichtung des Schlauches 21 geneigt angeordnet, was dessen Einschieben in den Schlauchverbinder 1 begünstigt.

Darüberhinaus weisen die Ringwulste 25 an ihrer patientenfernen Seite PF ebenfalls eine Neigung entsprechend einem Winkel α₂ von 10 bis 45 Grad auf, wodurch sich widerhakenartige Schultern 26 bilden, welche sich bei der Dekonnektion in die Oberfläche 27 des Saugschlauches 21 eingraben, so daß eine erhebliche Kraft ZK erforderlich wird, um den Saugschlauch 21 zu entfernen. Ein unbeabsichtigtes Lösen der Verbindung wird ausgeschlossen, da sich bei Einleitung der Dekonnektionszugkraft ZK die Ringwulste 25 um einen Betrag α' aufrichten, wodurch der Formschluß erhöht wird.

Die Höhe h der Ringwulste 25 kann hierbei mit 0,1 bis 0,2 mm sehr niedrig sein und ist nur zum besseren Verständnis vergrößert und unmaßstäblich dargestellt.

Der Schlauchverbinder 31 der Figuren 5 und 6 besteht ebenfalls aus sieben Kupplungsabschnitten 32 bis 38, welche durch Nuten 9 zueinander begrenzt bzw. dort z. B. bei S2-S2 voneinander durch Schnitt getrennt werden können.

Jedem Kupplungsabschnitt 32 bis 38 ist ein konischer Bohrungsabschnitt 42 bis 48 zugeordnet und zwar dermaßen, daß sich deren Lumen von der patientennahen Seite PN bis zur patientenfernen Seite PF stufenförmig erweitert.

Der patientenferne Saugschlauch 22 wird wieder, wie oben bereits beschrieben, im konischen Bohrungsabschnitt 48 auf bekannte Weise befestigt.

Der patientennahe Saugschlauch 21 ist auch hier je nach hydraulischen Erfordernissen in seinem Durchmesser wählbar und wird in den dazugehörigen Bohrungsabschnitt 42 bis 47 hier in Fig. 6 z. b. in den konischen Bohrungsabschnitt 46 eingeführt, nachdem die Abschnitte 32 bis 35 durch Schnitt bei S2-S2 entfernt wurden.

Durch die Konizität der Bohrungsabschnitte 42 bis 47 läßt sich der Saugschlauch relativ leicht in den Schlauchverbinder 31 einführen. Er wird über die Schulter 51 hinaus eingeschoben, so daß sich das Schlauchende 21a wieder erweitert. Bei der Dekonnektion ist eine erhebliche Zugkraft ZK erforderlich, da sich die Schulter 51 widerhakenartig und formschlußverstärkend auf die Oberfläche 27a des expandierten Schlauchendes 21 a auswirkt, wodurch auch hier ein unbeabsichtigtes Lösen der Schlauchverbindung 21/22 ausgeschlossen wird.

Der Schlauchverbinder 61 nach Fig. 7 bis 9 besteht aus sechs Kupplungsabschnitten 62 bis 67, welche wiederum durch Nuten 9 begrenzt sind. Jedem Kupplungsabschnitt 62 bis 67 ist ein Bohrungsabschnitt 72 bis 77 zur Aufnahme der Saugschläuche 21, 22 zugeordnet. Dabei sind die Bohrungsabschnitte 72, 73, 74 zylindrisch ausgeführt und weisen, wie im Beispiel nach Fig. 1 bis 4, umlaufende Ringwulste 25 auf. Die Bohrungsabschnitte 75 und 76 jedoch sind konisch wie im Beispiel nach Fig. 5 und 6 ausgeführt. Somit läßt sich dieser Schlauchverbinder sowohl für kleine als auch größere Saugschläuche verwenden.

Die Fig. 8 zeigt die Verwendung des bei S3-S3 abgeschnittenen Schlauchverbinders 61 für kleine Saugschläuche 21, wobei der patientenferne Saugschlauch 22, wie vorweg beschrieben, in einer konischen Bohrung 77 befestigt ist.

Die Fig. 9 zeigt die Verwendung des bei S4-S4 abgeschnittenen Schlauchverbinders 61 für größere Saugschläuche 21.

Die Anzahl der jeweiligen Kupplungs- und Bohrungsabschnitte kann natürlich geringer oder größer sein als in den Beispielen dargestellt. Entsprechendes gilt für die Lumina und der Verhältnisse zueinander.

## Patentansprüche

1. Schlauchverbinder aus gummielastischem Material zum Verbinden eines Schlauches mit in den Schlauchverbinder einzusteckenden dünneren Schläuchen unterschiedlichen Durchmessers mit abtrennbaren Kupplungsabschnitten mit stufenförmig sich erweiterndem zylindrischen Lumen,
**dadurch gekennzeichnet,**
**dass** die Lumina der Kupplungsabschnitte (2-8) mindestens einen Ringwulst (25) aufweisen oder die Lumina der Kupplungsabschnitte (32-38) über die gesamte Abschnittslänge als Konus gestaltet sind.

2. Schlauchverbinder nach Anspruch 1, **gekennzeichnet durch** eine zusammenhängende erste Gruppe von Kupplungsabschnitten mit zylinderförmigem Lumen mit jeweils mindestens einem Ringwulst (25) und eine zusammenhängende zweite Gruppe von Kupplungsabschnitten, deren Lumina konisch gestaltet sind.

3. Schlauchverbinder nach Anspruch 2, **dadurch gekennzeichnet, dass** die Lumina der Kupplungsabschnitte mit konischer Gestaltung größer sind als die Lumina der Kupplungsabschnitte mit zylinderförmigem Lumen.

4. Schlauchverbinder nach einem der Ansprüche 1-3, **dadurch gekennzeichnet, dass** die Ringwülste (25) in Einschubrichtung des Schlauches (21) geneigt angeordnet sind.

## Claims

1. A hose connector of rubber-elastic material for connecting a hose with thinner hoses that are to be set into the hose connector and have different diameters, comprising separable coupling sections with step-like flaring cylindrical lumen,
**characterized in**
**that** the lumina of the coupling sections (2-8) have at least one annular bead (25) or the lumina of the coupling sections (32-38) are of conical configuration over the entire length of the section.

2. The hose connector of claim 1, **characterized by** a contiguous first set of coupling sections with cylindrical lumen having at least one annular bead (25), and a contiguous second set of coupling sections whose lumina are conically shaped.

3. The hose connector of claim 2, **characterized in that** the lumina of the conically shaped coupling sections are larger than the lumina of the coupling sections with cylindrical lumen.

4. The hose connector of one of claims 1-3, **characterized in that** the annular beads (25) are inclined in the direction of insertion of the hose.

## Revendications

1. Raccord de tubulures ou tuyaux souples, en un matériau élastique du type caoutchouc, destiné à relier une tubulure ou un tuyau souple à des tubulures ou tuyaux souples plus fins, de différents diamètres et devant être insérés dans le raccord de tubulures ou tuyaux souples, qui comprend des tronçons de raccord présentant une lumière cylindrique s'évasant de manière étagée, **caractérisé en ce que** les lumières des tronçons de raccord (2-8) présentent au moins un bourrelet annulaire (25), ou bien les lumières des tronçons de raccord (32-38) sont réalisées sous forme de cône sur toute la longueur du tronçon.

2. Raccord de tubulures ou tuyaux souples selon la revendication 1, **caractérisé par** un premier groupe continu de tronçons de raccord à lumière de forme cylindrique comprenant chacun au moins un bourrelet annulaire (25), et un second groupe continu de tronçons de raccord dont les lumières sont de configuration conique.

3. Raccord de tubulures ou tuyaux souples selon la revendication 2, **caractérisé en ce que** les lumières des tronçons de raccord de configuration conique, sont plus grandes que les lumières des tronçons de raccord à lumière de forme cylindrique.

4. Raccord de tubulures ou tuyaux souples selon l'une des revendications 1 à 3, **caractérisé en ce que** les bourrelets annulaires (25) sont disposés de manière inclinée dans la direction d'insertion de la tubulure ou du tuyau flexible (21).
